# EUROPEAN PATENT APPLICATION

(11) **EP 1 075 835 A1**
(43) Date of publication of application: **14.02.2001**
(21) Application number: 00117013.3
(22) Date of filing: 08.08.2000
(51) Int. Cl.: A61K 7/48

(54) **Use of aminodiol derivatives in skin cosmeics**

(30) Priority: 09.08.1999 JP 22507999; 25.04.2000 JP 2000124198
(71) Applicant: SHISEIDO COMPANY LIMITED, Chuo-ku, Tokyo 104-8010 (JP)
(72) Inventor: Maeda, Kazuhisa, c/o Shiseido Research Center (1), Yokohama-shi, Kanagawa 223-8553 (JP); Katsuta, Yuji, c/o Shiseido Research Center (1), Yokohama-shi, Kanagawa 223-8553 (JP)
(74) Representative: Henkel, Feiler, Hänzel

(57) **Abstract**

To provide an external-use composition which exhibits excellent effect of improving decrease in turnover rate of the keratinous layer caused by aging; more specifically, retarding skin aging as well as preventing unfavorable skin conditions of various types such as spots, dull appearance, wrinkles, and skin-roughening. The external-use composition contains a propanediol derivative represented by formula (I): 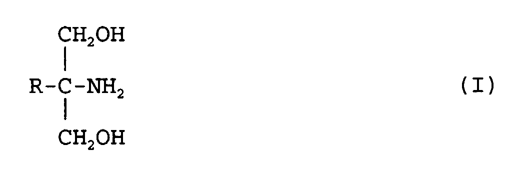 (wherein R represents a hydrogen atom, a methyl group, or an ethyl group) or a pharmaceutically acceptable salt thereof.

## Description

### BACKGROUND OF THE INVENTION

### Field of the Invention

The present invention relates to a composition for external use, and more particularly to a composition for external use exhibiting, among others, an effect of retarding aging of the skin.

### Background Art

Efforts towards discovery of an ingredient which is effective for treatment of various types of unfavorable skin conditions and provision of improved compositions for external use (hereinafter referred to as simply "external-use compositions") by incorporating such an ingredient into existing compositions have always been recognized as valuable development activities.

In addition to typical unfavorable skin conditions such as spots, dull appearance, wrinkles, and roughening of the skin, the concept "skin aging" has recently become of interest. "Skin aging" refers to aging of the skin, accompanied by typical phenomena such as wrinkles, flabbiness, and spots. These phenomena progress with age, and other factors such as exposure to UV rays, smoking, insufficient sleep, and air pollution are known to accelerate these phenomena.

In the healthy epidermis, generation of new cells in the basal layer and falling of old keratinocytes from the keratinous layer are equilibrated. When the equilibrium is lost and the keratinous layer is thickened by slowed falling of old keratinocytes, the skin may lose its shiny appearance or may become chapped, as is usually observed in winter. In addition, the turnover rate of the keratinous layer decreases with age, thereby retarding metabolism in the skin and permitting spots and wrinkles to become conspicuous.

J. Koyama *et al*. have already reported, in *Fragrance Journal* 1, 13-18, 1995, that thickening of the keratinous layer induced by decrease in turnover rate of the keratinous layer is positively correlated with decrease in desmosome decomposition activity (chymotrypsin-like enzyme activity), and that decrease in water content of the keratinous layer reduces enzyme activity.

Compounds such as α-hydroxy acid (AHA) and vitamin A derivatives are known as cosmetic agents which can improve the slowed turnover rate of the keratinous layer accompanying retarding metabolism in the skin caused by aging. E. J. Van Scott *et al*. have reported, in *Int*. *J*. *Dermatol*. 26, 90, 1987 and *Skin* & *Allergy News* 18, 38, 1987, that AHA and vitamin A derivatives reduce formation of wrinkles and improve dry skin, acne, and senile pigmented spots. Thus, these compounds have been incorporated into a variety of cosmetics. AHA smoothes the skin, and the mechanism thereof is considered to involve loosening adhesion between keratinocytes and preventing stratification of keratinocytes. Vitamin A derivatives exert effects on equilibrium between proliferation and differentiation of epidermal cells, thereby possibly preventing stratification of keratinocytes and activating metabolism in the skin.

However, AHA and vitamin A derivatives themselves are not mild to the skin, and therefore, not only are their incorporation amounts limited, but care must be exerted during manufacture of various forms of compositions. At present, insufficient effects are obtained from these compounds.

As mentioned above, desmosomes, which participate in intercellular adhesion, are known to play an important role in falling of old keratinocytes and activating metabolism in the skin. In fact, A. Lundstrom *et al*. reported in *J*. *Invest*. *Dermatol*., 91, 216-220, 1990 that acceleration of decomposition of desmoglein, serving as a protein constituting desmosomes, results in acceleration of falling of the keratinous layer.

In an attempt to solve the aforementioned problem, the present invention is to provide means for retarding skin aging as well as preventing unfavorable skin conditions of various types, particularly by use of an ingredient other than AHA and vitamin A derivatives which accelerates decomposition of desmoglein.

### SUMMARY OF THE INVENTION

The present inventors have carried out extensive research regarding the desmoglein decomposition effect of a variety of compounds, in search for a novel keratious layer-exfoliating ingredient of high effect, and have found that specific propanediol derivatives exhibit an excellent effect of improving decrease in turnover rate of the keratinous layer caused by aging; more specifically, retarding skin aging as well as preventing unfavorable skin conditions of various types, such as spots, dull appearance, wrinkles, and skin-roughening. The present invention has been accomplished on the basis of this finding.

Accordingly, the present invention provides an external-use composition containing a propanediol derivative represented by formula (I): 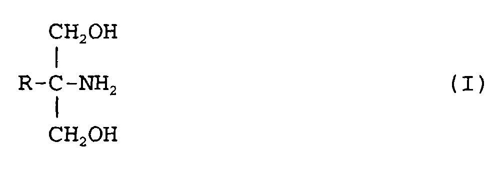 (wherein R represents a hydrogen atom, a methyl group, or an ethyl group) or a pharmaceutically acceptable salt thereof.

Typically, the propanediol derivative represented by formula (I) is 2-amino-1,3-propanediol or 2-amino-2-methyl-1,3-propanediol and their salts are hydrochloric acid salt, sulfuric acid salt or oxalic acid salt.

The amount of the propanediol derivative represented by formula (I) in the external-use composition is preferably 0.005-20.0 wt.%, more preferably 0.01-10.0 wt.%.

Also in the present invention, as mentioned above, the term "skin aging" refers to skin aging caused by aging, exposure to UV rays, smoking, insufficient sleep, and air pollution. Specifically, skin aging is retarded by controlling a variety of aging phenomena such as spots, dull appearance, wrinkles, and skin-roughening. Thus, an effect of retarding aging of the skin is a wide-ranging effect. The external-use composition of the present invention is employed for preventing specific unfavorable skin conditions such as spots, dull appearance, wrinkles, and skin-roughening, whereas "retarding skin aging" is not limited to prevention of these specific phenomena.

### BRIEF DESCRIPTION OF THE DRAWINGS

Various other objects, features, and many of the attendant advantages of the present invention will be readily appreciated as the same becomes better understood with reference to the following detailed description of the preferred embodiments when considered in connection with accompanying drawings, in which:
Fig. 1 is a graph showing comparison of desmoglein decomposition effect of the compositions using 2-amino-1,3-propanediol and its salt of the present invention and that of a comparative composition;
Fig. 2 is a graph showing comparison of desmoglein decomposition effect of the compositions using 2-amino-2-methyl-1,3-propandiol and its salt of the present invention and that of a comparative composition;
Fig. 3 is a graph showing comparison of effect on the turnover rate of the keratinous layer provided by the compositions using 2-amino-1,3-propanediol and its salt of the present invention and that provided by a comparative composition;
Fig. 4 is a graph showing comparison of effect on water content of the keratinous layer provided by the compositions using 2-amino-1,3-propanediol and its salt of the present invention and that provided by a comparative composition;
Fig. 5 is a graph showing comparison of effect on elasticity of the skin provided by the compositions of the present invention using 2-amino-1,3-propanediol and its salt and that provided by a comparative composition;
Fig. 6 is a graph showing comparison of effect on the turnover rate of the keratinous layer provided by the compositions using 2-amino-2-methyl-1,3-propandiol and its salt of the present invention and that provided by a comparative composition;
Fig. 7 is a graph showing comparison of effect on water content of the keratinous layer provided by the compositions using 2-amino-2-methyl-1,3-propandiol and its salt of the present invention and that provided by a comparative composition; and
Fig. 8 is a graph showing comparison of effect on elasticity of the skin provided by the compositions using 2-amino-2-methyl-1,3-propandiol and its salt of the present invention and that provided by a comparative composition.

### DETAILED DESCRIPTION OF PREFERRED EMBODIMENTS

Embodiments for carrying out the present invention will next be described.

### A. Active ingredients of the composition of the invention:

The active ingredient of the external-use composition is a propanediol derivative represented by formula (I) (hereinafter may be referred to as propanediol derivative (I)) or a pharmaceutically acceptable salt thereof.

The propanediol derivative is a dihydric alcohol represented by formula (I). Specifically, when R is a hydrogen atom, the derivative is 2-amino-1,3-propanediol. Similarly, when R is a methyl group, the derivative is 2-amino-2-methyl-1,3-propanediol, whereas when R is an ethyl group, the derivative is 2-amino-2-ethyl-1,3-propanediol.

These three compounds; i.e., 2-amino-1,3-propanediol, 2-amino-2-methyl-1,3-propanediol, and 2-amino-2-ethyl-1,3-propanediol, can be produced through a conventional method. As systhesis of 2-amino-2-methyl-1,3-propandiol, nitroethane obtained by nitration of natural gas reacts with formaldehyde in the presence of an alkaline catalyst, to give 2-nitro-2-methyl-1,3-propanediol. The intermediate is reduced to give target compound. The commercial products are also available.

These propanediol derivatives and their salts are known compounds. 2-Amino-2-methyl-1,3-propanediol is just known as using a pH-adjusting agent or an emulsifier in cosmetics, however, the pharmacological effect of these three compounds on the skin has never been reported.

We have found the propanediol derivative (I) or a salt thereof exhibits an effect of retarding aging of the skin; i.e., the compounds accelerate the turnover rate of the keratinous layer lowered by aging and activate metabolism in the skin, thereby attaining skin conditions of much suppleness and bright appearance without spots, dull appearance, wrinkles, or skin-roughening.

The mechanism of retarding of skin aging by propanediol derivative (I) or a salt thereof has never been reported. We have found the propanediol derivative (I) or a salt thereof promotes decomposition of desmosomes participating in intercellular adhesion in the keratinous layer; exhibits excellent effect of accelerating the turnover rate of the keratinous layer in the aged skin; and restores elasticity of the skin. We have, first of all, thought that the dihydric alcohol or a salt thereof accelerates the turnover rate of the keratinous layer in the aged skin and improve conditions of the keratinous layer, thereby activating metabolism in the skin and exhibiting an effect of retarding aging of the skin.

The propanediol derivative (I) or a salt thereof may be incorporated singly or in combination of two or more species into the composition of the present invention. The amount of the compounds is preferably 0.005-20.0 wt.% based on the entire amount of the composition, more preferably 0.01-10.0 wt.%. When the amount is less than 0.005 wt.%, the effect of retarding aging of the skin is poor, whereas when the amount is in excess of 20.0 wt.%, the effect commensurate with the amount of addition is not attained.

### B. Embodiments of the composition of the invention:

As described above, the present invention provides an external-use composition incorporating the aforementioned propanediol derivative (I) or a salt thereof, which composition exhibits effects of retarding skin aging and preventing spots, dull appearance, wrinkles, and skin-roughening.

In addition to the above ingredients, other ingredients which are typically employed in cosmetics and medical preparations for external use may appropriately be incorporated into the composition of the present invention in accordance with needs. Examples of additive ingredients include aqueous ingredients, oil ingredients, powder ingredients, alcohols other than the propanediol derivatives, humectants, thickeners, UV-absorbents, whitening agents, preservatives, anti-oxidants, surfactants, perfumes, colorants, and nutrients for the skin.

The composition of the present invention may be applied to a cosmetic, drug, or quasi-drug. The composition may be formed into a variety of dosage forms and product forms, such as ointment, cream, milky lotion, lotion, pack, and bath-water additive.

### EXAMPLES

The present invention is described in detail by examples, which should not be construed as limiting the present invention thereto. Throughout the following examples, the amount of an ingredient incorporated into a composition is represented by wt.% based on the composition.

### Test Example 1 Evaluation for promotion of decomposition of desmoglein (1)

To both sides of a sheet (1.5 ± 0.2 mg) of the keratinous layer was applied 2-amino-1,3-propanediol, 2-amino-1,3-propanediol hydrochloride, or glycolic acid (5 µL for each side). Glycolic acid, which is one of AHA's, was used as a positive control. The sheet was sandwiched by stainless steel mesh sheets and allowed to stand at 37°C with a humidity of 75% for one week. Subsequently, the sheet was transferred into a microtube, and an extracting liquid (100 µL) was added to the microtube. The mixture was maintained at 37°C overnight to extract protein from the mixture. The extract was subjected to electrophoresis by use of agarose gel, and the gel was transferred to a PVDF membrane. An anti-desmoglein antibody and an alkaline-phosphatase-labeled anti-IgG sequentially reacted with the gel, and the resultant gel was colored with alkaline phosphatase. The image of bands formed on the membrane was captured by an image-scanner, and was analyzed, to obtain the amount of desmoglein. The test was performed twice, and an average value of the amount was calculated. The average values of samples were compared.

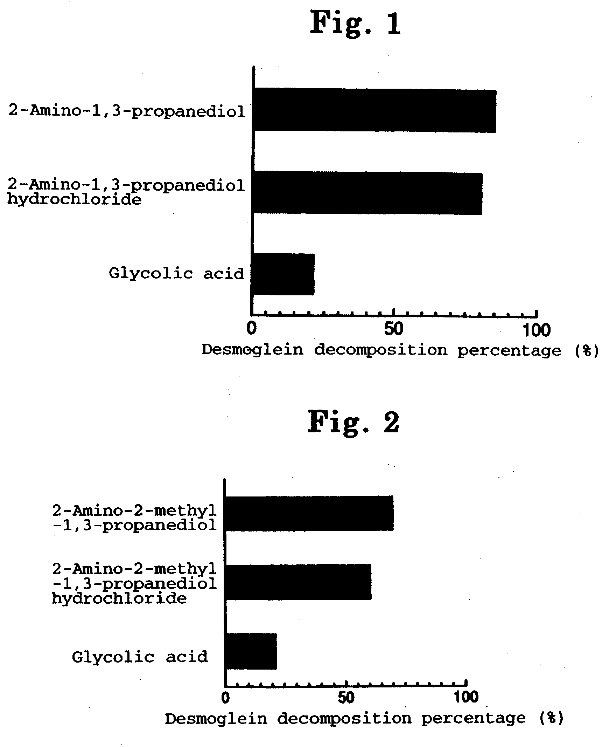

As shown in Fig. 1, 2-amino-1,3-propanediol and 2-amino-1,3-propanediol hydrochloride were found to exhibit a stronger effect of promoting decomposition of desmoglein than does glycolic acid as a positive control.

### Test Example 2 Evaluation for promotion of decomposition of desmoglein (2)

The effect of 2-amino-2-methyl-1,3-propanediol and its hydrochloride was investigated in accordance with the same method.

As shown in Fig. 2, 2-amino-2-methyl-1,3-propanediol and its hydrochloride were also found to exhibit a stronger effect of promoting decomposition of desmoglein than does glycolic acid as a positive control.

### Test Example 3 Evaluation for mitigation of decrease in turnover rate of the keratinous layer induced by aging (1)

The effect of 2-amino-1,3-propanediol and 2-amino-1,3-propanediol hydrochloride on mitigation of decrease in turnover rate of the keratinous layer induced by aging was investigated in four middle-aged subjects. The turnover rate, water content of the keratinous layer, and elasticity of the skin were evaluated.

1% Aqueous solutions of 2-amino-1,3-propanediol and its hydrochloride were employed as the representative compositions of the present invention. A 1% aqueous solution of glycolic acid―which is one type of AHA―was employed as a comparative composition, and water (purified water) was employed as a reference.

A 1% phosphor solution (1 mL) in ethanol was added to an outer syringe (length: about 2 cm) of a 10-mL disposable injector, to bring the solution into contact with the skin for one minute (four portions on the inside of the forearm). From the next day, each sample including water was applied to the phosphor-contacted portions. Application was performed three times per day and continued for four consecutive days.

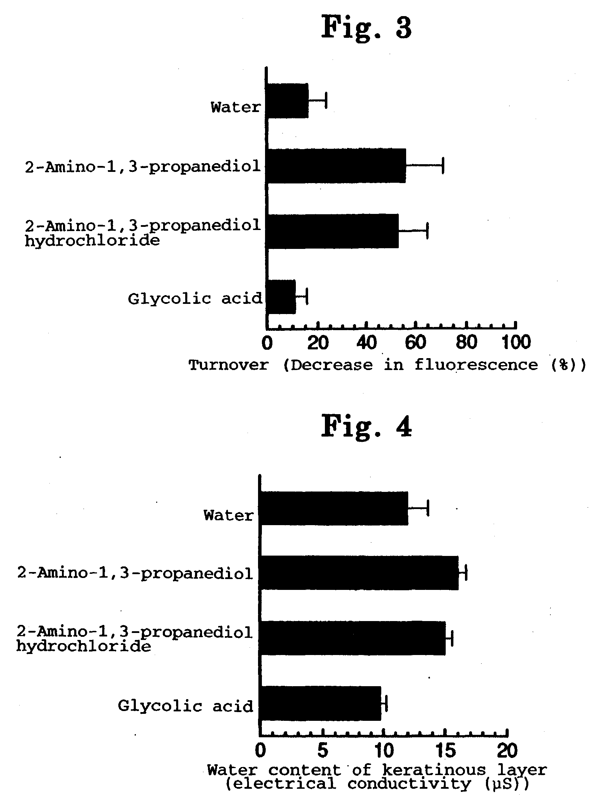

On the fifth day after the test was initiated, intensity of fluorescence from the skin surface was measured by use of a fluorescence-detector designed for this particular use. Decrease in fluorescence was employed as an index of the turnover rate of the keratinous layer. The larger the decrease in fluorescence, the greater the turnover rate. The results are shown in Fig. 3.

Water content of the keratinous layer was measured by use of an apparatus for measuring electrical conductivity (Skicon-200, product of IBS). The conductivity was employed as an index of water content. Thus, the higher the water content, the higher the electrical conductivity. The results are shown in Fig. 4.

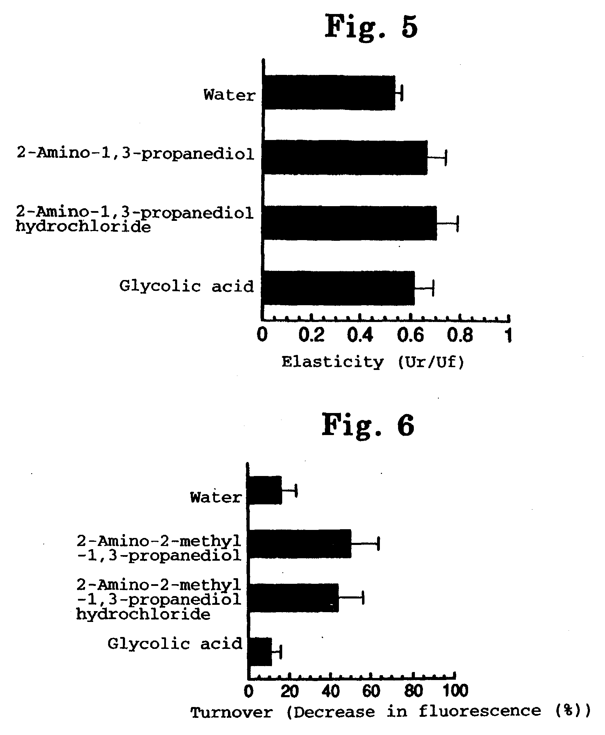

Elasticity of the skin was measured by use of Cutometer SEM474 (product of Courage + Khazaka). The elasticity was obtained from extension (Uf) and retraction (Ur) of the skin when a negative pressure of 300 hPa was repeatedly applied to the skin by use of a probe having a diameter of 2 mm. The ratio (Ur/Uf) was employed as an index of elasticity of the skin. The higher the ratio, the higher the elasticity of the skin. The results are shown in Fig. 5.

As is clear from Figs. 3 to 5, 2-amino-1,3-propanediol and its hydrochloride, which are employed as ingredients of the composition of the present invention, mitigate decrease in turnover rate induced by aging (Fig. 3) and improve water content of the keratinous layer (Fig. 4) and elasticity of the skin (Fig. 5).

These ingredients were found to exhibit the above three effects more strongly than does glycolic acid, which is known as a turnover-promoter of the keratinous layer.

### Test Example 4 Evaluation for mitigation of decrease in turnover rate of the keratinous layer induced by aging (2)

The turnover rate, water content of keratinous layer and elasticity of the skin in case of 2-amino-2-methyl-1,3-propanediol and its hydrochloride were also investigated.

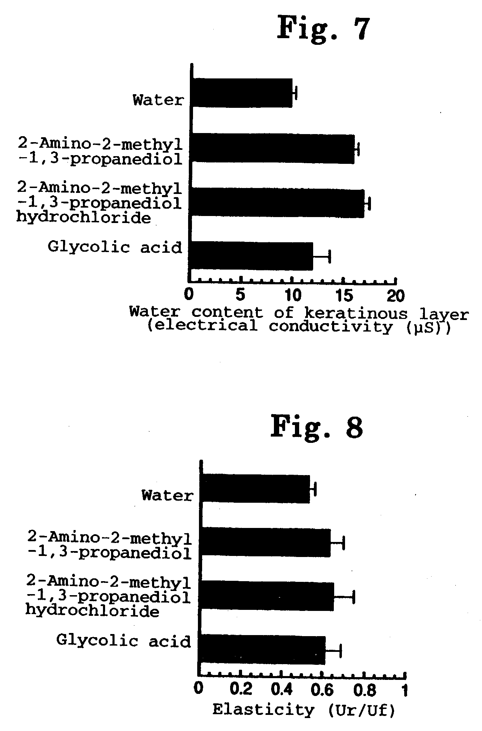

As is clear from the turnover rate induced by aging (Fig. 6), water content of the keratinous layer (Fig. 7) and elasticity of the skin (Fig. 8), 2-amino-2-methyl-1,3-propanediol and its hydrochloride were found to exhibit the above three effects more strongly than does glycolic acid.

### Test Example 5 Evaluation for improving skin conditions by use of the composition of the invention (actual use over a continuous four-week period) (1)

The external-use composition of the present invention was applied to women complaining spots, dull appearance, wrinkles, and skin-roughening. Improvement in skin conditions was evaluated.

Specifically, 40 women panelists suffering from spots, dull appearance, wrinkles, and skin-roughening were enrolled in the test. One lotion according to the present invention was applied to one cheek of each panelist, and one comparative lotion was applied to the other cheek. The compositions (amount based on wt.%) of the lotions employed in the test are shown in Table 1. Application was performed twice per day and continued for consecutive four weeks. Skin conditions were observed by the naked eye, and evaluated on the basis of the following standards.

The results are shown in Table 2.

### [Standards for evaluation]

### Evaluation of efficacy of the composition by visual observation

- Standards for evaluating improvement effect on spots and dull appearance
   Remarkably effective : Unfavorable conditions disappeared
   Effective : Unfavorable conditions were improved
   Slightly effective : Unfavorable conditions were slightly improved
   No effect : No improvement in unfavorable conditions
- Standards for evaluating improvement effect on wrinkles
   Remarkably effective : Unfavorable conditions disappeared
   Effective : Unfavorable conditions were improved
   Slightly effective : Unfavorable conditions were slightly improved
   No effect : No improvement in unfavorable conditions
- Standards for evaluating improvement effect on skin-roughening
   Remarkably effective : Unfavorable conditions disappeared
   Effective : Unfavorable conditions were improved
   Slightly effective : Unfavorable conditions were slightly improved
   No effect : No improvement in unfavorable conditions

### Evaluation of improvement effect

AA : 80% or more (efficacy) of the subjects were categorized as "Remarkably effective," Effective," or "Slightly effective."
BB : 50-80% (efficacy) of the subjects were categorized as "Remarkably effective," Effective," or "Slightly effective."
CC : 30-50% (efficacy) of the subjects were categorized as "Remarkably effective," Effective," or "Slightly effective."
DD : Less than 30% (efficacy) of the subjects were categorized as "Remarkably effective," Effective," or "Slightly effective."

**Table 1**

| Ingredients | Example 1 | Example 2 | Comp. Ex. 1 | Comp. Ex. 2 |
|---|---|---|---|---|
| 2-Amino-1,3-propanediol | 1 0 | - | - | - |
| 2-Amino-1,3-propanediol HCl | - | 1.0 | - | - |
| Glycolic acid | - | - | 1.0 | - |
| Glycerin | 1.0 | 1.0 | 1.0 | 1.0 |
| 1,3-Butylene glycol | 4.0 | 4.0 | 4.0 | 4.0 |
| Ethanol | 7.0 | 7.0 | 7.0 | 7.0 |
| POE (20 mol) oleyl alcohol | 0.5 | 0.5 | 0.5 | 0.5 |
| Purified water | balance | balance | balance | balance |

**Table 2**

| Samples | Example 1 | Example 2 | Comp. Ex. 1 | Comp. Ex. 2 |
|---|---|---|---|---|
| Spot, dull appearance | BB | BB | CC | DD |
| Wrinkles | BB | BB | CC | DD |
| Skin-roughening | AA | AA | BB | DD |

As is clear from Table 2, the composition containing 2-amino-1,3-propanediol (Example 1) and the composition containing its hydrochloride (Example 2) exhibit excellent effects of improving spots, dull appearance, wrinkles, and skin-roughening as compared with the composition containing glycolic acid (Comp. Ex. 1) or the active-ingredient-free composition (Comp. Ex. 2). Particularly, these two compositions according to the present invention exhibit higher efficacy in this test than does the composition containing glycolic acid, which is known to improve spots, dull appearance, wrinkles, and skin-roughening by accelerating the turnover rate of the keratinous layer. The results indicate that the external-use composition of the present invention is highly effective for improving spots, dull appearance, wrinkles, and skin-roughening.

### Test Example 6 Evaluation for improving skin conditions by use of the composition of the invention (actual use over a continuous four-week period) (2)

The investigation of 2-amino-2-methyl-1,3-propanediol and its hydrochloride was made as the same procedure.

Specifically, 40 women panelists suffering from spots, dull appearance, wrinkles, and skin-roughening were enrolled in the test. One lotion according to the present invention was applied to one cheek of each panelist, and one comparative lotion was applied to the other cheek. The compositions (amount based on wt.%) of the lotions employed in the test and the estimation are shown in Table 3. Application was performed twice per day and continued for four weeks. Skin conditions were observed by the naked eye, and evaluated on the basis of the standards described in Test Example 5.

**Table 3**

| Ingredients | Example 3 | Example 4 | Comp. Ex. 3 | Comp. Ex. 4 |
|---|---|---|---|---|
| 2-Amino-2-methyl-1,3-propanediol | 1.0 | - | - | - |
| 2-Amino-2-methyl-1,3-propanediol HCl | - | 1.0 | - | - |
| Glycolic acid | - | - | 1.0 | - |
| Glycerin | 1.0 | 1.0 | 1.0 | 1.0 |
| 1,3-Butylene glycol | 4.0 | 4.0 | 4.0 | 4.0 |
| Ethanol | 7.0 | 7.0 | 7.0 | 7.0 |
| POE (20 mol) oleyl alcohol | 0.5 | 0.5 | 0.5 | 0.5 |
| Purified water | Balance | balance | balance | balance |

| Estimation | | | | |
|---|---|---|---|---|
| Spot, dull appearance | BB | BB | CC | DD |
| Wrinkles | BB | BB | CC | DD |
| Skin-roughening | AA | AA | BB | DD |

As is clear from Table 3, the composition containing 2-amino-2-methyl-1,3-propanediol (Example 3) and the composition containing its hydrochloride (Example 4) exhibit excellent effects of improving spots, dull appearance, wrinkles, and skin-roughening as compared with the comparative cases.

Thus, pharmacological effect of 2-amino-1,3-propanediol and its salts and 2-amino-2-methyl-1,3-propanediol and its salts on desmoglein decomposition, turnover rate of the keratinous layer, water content of the keratinous layer, and elasticity of the skin was elucidated. Accordingly, the same pharmacological effect of 2-amino-2-ethyl-1,3-propanediol and its salts can be easily expected.

Formulation examples of the external-use composition of the present invention was described. All of the following compositions according to the invention have been tested as described above and found to be highly effective for improving spots, dull appearance, wrinkles, and skin-roughening and retarding skin aging.

| [Formulation Example 1] | Cream |
|---|---|
| Ingredient | Amount (wt.%) |
| stearic acid | 5.0 |
| stearyl alcohol | 4.0 |
| isopropyl myristate | 18.0 |
| glyceryl monostearate | 3.0 |
| propylene glycol | 10.0 |
| 2-amino-1,3-propanediol hydrochloride | 0.2 |
| sodium bisulfite | 0.01 |
| preservative | suitable amount |
| perfume | suitable amount |
| purified water | balance |

### 〈Preparation Method〉

Propylene glycol and 2-amino-1,3-propanediol hydrochloride were added to the purified water and the mixture was maintained at 70°C (aqueous phase). The remaining ingredients were mixed, and the mixture was melted and maintained at 70°C (oily phase). The oily phase was gradually added to the aqueous phase. After completion of the addition, the mixture was maintained at the same temperature for a while and was homogeneously emulsified by use of a homogenizer, and was cooled to 30°C under sufficient stirring to obtain a cream.

| [Formulation Example 2] | Cream |
|---|---|
| Ingredient | Amount (wt.%) |
| stearic acid | 2.0 |
| stearyl alcohol | 7.0 |
| hydrogenated lanolin | 2.0 |
| squalane | 5.0 |
| 2-octyldodecyl alcohol | 6.0 |
| POE* (25 mol) cetyl ether | 3.0 |
| glyceryl monostearate | 2.0 |
| propylene glycol | 5.0 |
| 2-amino-1,3-propanediol | 0.5 |
| citric acid | 0.7 |
| ethyl paraben | 0.3 |
| perfume | suitable amount |
| purified water | balance |

| | |
|---|---|
| *POE: polyoxyethylene | |

### 〈Preparation Method〉

The mixture of 2-amino-1,3-propanediol, citric acid, and propylene glycol was added to the purified water. The mixture was maintained at 70°C (aqueous phase). The remaining ingredients were mixed, and the mixture was melted and maintained at 70°C (oily phase). The oily phase was gradually added to the aqueous phase for preliminary emulsification. Subsequently, the mixture was homogeneously emulsified by use of a homogenizer, and was cooled to 30°C under sufficient stirring to obtain a cream.

| [Formulation Example 3] | Cream |
|---|---|
| Ingredient | Amount (wt.%) |
| solid paraffin | 5.0 |
| beeswax | 10.0 |
| Vaseline | 15.0 |
| liquid paraffin | 41.0 |
| glyceryl monostearate | 2.0 |
| POE (20 mol) sorbitan monolaurate | 2.0 |
| powdery soap | 0.1 |
| borax | 0.2 |
| 2-amino-1,3-propanediol hydrochloride | 3.0 |
| ethyl paraben | 0.3 |
| perfume | suitable amount |
| purified water | balance |

### 〈Preparation Method〉

The mixture of 2-amino-1,3-propanediol hydrochloride, powdery soap, and borax was added to the purified water. The mixture was maintained at 70°C (aqueous phase). The remaining ingredients were mixed, and the mixture was melted and maintained at 70°C (oily phase). The oily phase was gradually added to the aqueous phase with stirring for reaction. Subsequently, the mixture was homogeneously emulsified by use of a homogenizer, and was cooled to 30°C under sufficient stirring.

| [Formulation Example 4] | Milky Lotion |
|---|---|
| Ingredient | Amount (wt.%) |
| stearic acid | 2.5 |
| cetyl alcohol | 1.5 |
| Vaseline | 5.0 |
| liquid paraffin | 10.0 |
| POE (10 mol) monooleate | 2.0 |
| polyethylene glycol 1500 | 3.0 |
| triethanolamine | 1.0 |
| carboxyvinyl polymer (trade name: Carbopole 941, B. F. Goodrich Chemical Company) | 0.05 |
| 2-amino-1,3-propanediol oxalate | 0.02 |
| ethyl paraben | 0.3 |
| perfume | suitable amount |
| purified water | balance |

### 〈Preparation Method〉

The carboxyvinyl polymer was added to a small amount of the purified water, to obtain a solution (phase A). The mixture of polyethylene glycol 1500, triethanolamine, and 2-amino-1,3-propanethol oxalate was added to the remaining portion of the purified water. The mixture was heated for solution, and the solution was maintained at 70°C (aqueous phase). The remaining ingredients were mixed, and the mixture was melted and maintained at 70°C (oily phase). The oily phase was added to the aqueous phase for preliminary emulsification, and the phase A mixture was added thereto. The mixture was homogeneously emulsified by use of a homogenizer and was cooled to 30°C under sufficient stirring to obtain a milky lotion.

| [Formulation Example 5] | Milky Lotion |
|---|---|
| Ingredient | Amount (wt.%) |
| microcrystalline wax | 1.0 |
| beeswax | 2.0 |
| lanolin | 20.0 |
| liquid paraffin | 10.0 |
| squalane | 5.0 |
| sorbitan sesquioleate | 4.0 |
| POE (20 mol) sorbitan monooleate | 1.0 |
| propylene glycol | 7.0 |
| 2-amino-1,3-propanediol | 4.0 |
| glutamic acid | 0.7 |
| ethyl paraben | 0.3 |
| perfume | suitable amount |
| purified water | balance |

### 〈Preparation Method〉

The mixture of 2-amino-1,3-propanediol, glutamic acid, and propylene glycol was added to the purified water. The mixture was heated for solution, and the solution was maintained at 70°C (aqueous phase). The remaining ingredients were mixed, and the mixture was melted and maintained at 70°C (oily phase). The oily phase was gradually added to the aqueous phase, and the mixture was homogeneously emulsified by use of a homogenizer and was cooled to 30°C under sufficient stirring to obtain a milky lotion.

| [Formulation Example 6] | Jelly |
|---|---|
| Ingredient | Amount (wt.%) |
| 95% ethyl alcohol | 10.0 |
| dipropylene glycol | 15.0 |
| POE (50 mol) oleyl ether | 2.0 |
| carboxyvinyl polymer (trade name: Carbopole 940, B. F. Goodrich Chemical Company) | 0.05 |
| 2-amino-1,3-propanediol | 0.05 |
| gluconic acid | 0.1 |
| ethyl paraben | 0.3 |
| perfume | suitable amount |
| purified water | balance |

### 〈Preparation Method〉

Carbopole 940 was thoroughly dissolved in the purified water. The POE (50 mol) oleyl ether was added to the resultant aqueous phase. Subsequently, the aqueous phase was neutralized and thickened through addition of the 2-amino-1,3-propanediol, to which the remaining ingredients had been added in advance, to obtain a jelly.

| [Formulation Example 7] | Jelly |
|---|---|
| Ingredient | Amount (wt.%) |
| (phase A) | |
| 95% ethyl alcohol | 10.0 |
| POE (20 mol) octyldodecanol | 1.0 |
| pantothenyl ethyl ether | 0.1 |
| methyl paraben | 0.15 |

| (phase B) | |
|---|---|
| potassium hydroxide | 0.1 |

| (phase C) | |
|---|---|
| glycerin | 5.0 |
| dipropylene glycol | 10.0 |
| 2-amino-1,3-propanediol hydrochloride | 0.05 |
| carboxyvinyl polymer (trade name: Carbopole 940, B. F. Goodrich Chemical Company) | 0.2 |
| purified water | balance |

### 〈Preparation Method〉

Each of the "phase A" and the "phase C" ingredients were homogenously mixed. The "phase A" mixture was added to and dissolved in the "phase C" mixture. Thereafter, the "phase B" ingredient was added thereto and the mixture was charged into a container to obtain a jelly.

| [Formulation Example 8] | Pack |
|---|---|
| Ingredient | Amount (wt.%) |
| (phase A) | |
| dipropylene glycol | 5.0 |
| POE (60 mol) hydrogenated castor oil | 5.0 |

| (phase B) | |
|---|---|
| olive oil | 5.0 |
| tocopheryl acetate | 0.2 |
| ethyl paraben | 0.2 |
| perfume | 0.2 |

| (phase C) | |
|---|---|
| 2-amino-1,3-propanediol sulfate | 0.3 |
| polyvinyl alcohol (saponification value: 90; polymerization degree: 2,000) | 13.0 |
| ethyl alcohol | 7.0 |
| purified water | balance |

### 〈Preparation Method〉

Each of the "phase A", "phase B", and "phase C" ingredients were homogeously mixed. The "phase B" mixture was added to and dissolved in the "phase A" mixture. Subsequently, the "phase C" mixture was added thereto, and the mixture was charged into a container, to obtain a pack.

| [Formulation Example 9] | Solid-type foundation |
|---|---|
| Ingredient | Amount (wt.%) |
| talc | 43.1 |
| kaolin | 15.0 |
| sericite | 10.0 |
| zinc oxide | 7.0 |
| titanium dioxide | 3.8 |
| yellow iron oxide | 2.9 |
| black iron oxide | 0.2 |
| squalane | 8.0 |
| isostearic acid | 4.0 |
| POE sorbitan monooleate | 3.0 |
| isocetyl octanoate | 2.0 |
| 2-amino-1,3-propanediol | 0.2 |
| lactic acid | 0.5 |
| preservative | suitable amount |
| perfume | suitable amount |

### 〈Preparation Method〉

The powdery ingredients from the talc to the black iron oxide were sufficiently mixed by use of a blender. The oily ingredients from the squalane to the isocetyl octanoate, the 2-amino-1,3-propanediol, the lactic acid, the preservative, and the perfume were added to the resultant mixture. The mixture was thoroughly kneaded, charged into a container, and then shaped, to obtain a solid-type foundation.

| [Formulation Example 10] | Emulsion-type foundation |
|---|---|
| Ingredient | Amount (wt.%) |
| (powdery ingredients) | |
| titanium dioxide | 10.3 |
| sericite | 5.4 |
| kaolin | 3.0 |
| yellow iron oxide | 0.8 |
| red iron oxide | 0.3 |
| black iron oxide | 0.2 |

| (oily phase) | |
|---|---|
| decamethylcyclopentasiloxane | 11.5 |
| liquid paraffin | 4.5 |
| POE-modified dimethylpolysiloxane | 4.0 |

| (aqueous phase) | |
|---|---|
| purified water | 50.0 |
| 1,3-butylene glycol | 4.5 |
| 2-amino-1,3-propanediol | 1.0 |
| sorbitan sesquioleate | 3.0 |
| preservative | suitable amount |
| perfume | suitable amount |

### 〈Preparation Method〉

The aqueous phase ingredients were mixed and heated with stirring. The powdery ingredients which had been thoroughly mixed and pulverized were added thereto and subjected to processing in a homogenizer. The oily phase ingredients which had been heated and mixed were added, and the mixture was processed by use of a homogenizer. The perfume was added under stirring, followed by cooling to room temperature, to obtain an emulsion-type foundation.

| [Formulation Example 11] | Cream |
|---|---|
| Ingredient | Amount (wt.%) |
| stearic acid | 5.0 |
| stearyl alcohol | 4.0 |
| isopropyl myristate | 18.0 |
| glyceryl monostearate | 3.0 |
| propylene glycol | 10.0 |
| 2-amino-2-methyl-1,3-propanediol hydrochloride | 0.1 |
| sodium bisulfite | 0.01 |
| preservative | suitable amount |
| perfume | suitable amount |
| purified water | balance |

### 〈Preparation Method〉

The mixture of propylene glycol and 2-amino-2-methyl-1,3-propanediol hydrochloride was added to the purified water. The mixture was maintained at 70°C (aqueous phase). The remaining ingredients were mixed, and the resultant mixture was melted and maintained at 70°C (oily phase). The oily phase was gradually added to the aqueous phase. After completion of the addition, the mixture was maintained at the same temperature for a while. Thereafter, the mixture was homogeneously emulsified by use of a homogenizer, and the thus-obtained emulsion was cooled to 30°C under sufficient stirring, to obtain a cream.

| [Formulation Example 12] | Cream |
|---|---|
| Ingredient | Amount (wt.%) |
| stearic acid | 2.0 |
| stearyl alcohol | 7.0 |
| hydrogenated lanolin | 2.0 |
| squalane | 5.0 |
| 2-octyldodecyl alcohol | 6.0 |
| POE (25 mol) cetyl ether | 3.0 |
| glyceryl monostearate | 2.0 |
| propylene glycol | 5.0 |
| 2-amino-2-methyl-1,3-propanediol | 1.0 |
| citric acid | 0.7 |
| ethyl paraben | 0.3 |
| perfume | suitable amount |
| purified water | balance |

### 〈Preparation Method〉

The mixture of 2-amino-2-methyl-1,3-propanediol, citric acid, and propylene glycol was added to the purified water. The mixture was maintained at 70°C (aqueous phase). The remaining ingredients were mixed, and the resultant mixture was melted and maintained at 70°C (oily phase). The oily phase was gradually added to the aqueous phase for preliminary emulsification. Subsequently, the mixture was homogeneously emulsified by use of a homogenizer, and the thus-obtained emulsion was cooled to 30°C under sufficient stirring, to obtain a cream.

| [Formulation Example 13] | Cream |
|---|---|
| Ingredient | Amount (wt.%) |
| solid paraffin | 5.0 |
| beeswax | 10.0 |
| Vaseline | 15.0 |
| liquid paraffin | 41.0 |
| glyceryl monostearate | 2.0 |
| POE (20 mol) sorbitan monolaurate | 2.0 |
| powdery soap | 0.1 |
| borax | 0.2 |
| 2-amino-2-methyl-1,3-propanediol hydrochloride | 3.0 |
| ethyl paraben | 0.3 |
| perfume | suitable amount |
| purified water | balance |

### 〈Preparation Method〉

The mixture of 2-amino-2-methyl-1,3-propanediol hydrochloride, powdery soap, and borax was added to the purified water. The mixture was maintained at 70°C (aqueous phase). The remaining ingredients were mixed, and the mixture was maintained at 70°C (oily phase). The oily phase was gradually added to the aqueous phase with stirring for reaction. Subsequently, the mixture was homogeneously emulsified by use of a homogenizer, and was cooled to 30°C under sufficient stirring.

| [Formulation Example 14] | Milky Lotion |
|---|---|
| Ingredient | Amount (wt.%) |
| stearic acid | 2.5 |
| cetyl alcohol | 1.5 |
| Vaseline | 5.0 |
| liquid paraffin | 10.0 |
| POE (10 mol) monooleate | 2.0 |
| polyethylene glycol 1500 | 3.0 |
| triethanolamine | 1.0 |
| carboxyvinyl polymer (trade name: Carbopole 941, B. F. Goodrich Chemical Company) | 0.05 |
| 2-amino-2-methyl-1,3-propanediol oxalate | 0.01 |
| ethyl paraben | 0.3 |
| perfume | suitable amount |
| purified water | balance |

### 〈Preparation Method〉

The carboxyvinyl polymer was added to a small amount of the purified water to obtain a solution (phase A). The mixture of polyethylene glycol 1500, triethanolamine, and 2-amino-2-methyl-1,3-propanediol oxalate was added to the remaining portion of the purified water. The mixture was maintained at 70°C (aqueous phase). The remaining ingredients were mixed, and the mixture was maintained at 70°C (oily phase). The oily phase was added to the aqueous phase for preliminary emulsification, and the phase A mixture was added thereto. The mixture was homogeneously emulsified by use of a homogenizer, and after emulsification, the thus-obtained emulsion was cooled to 30°C under sufficient stirring to obtain a milky lotion.

| [Formulation Example 15] | Milky Lotion |
|---|---|
| Ingredient | Amount (wt.%) |
| microcrystalline wax | 1.0 |
| beeswax | 2.0 |
| lanolin | 20.0 |
| liquid paraffin | 10.0 |
| squalane | 5.0 |
| sorbitan sesquioleate | 4.0 |
| POE (20 mol) sorbitan monooleate | 1.0 |
| propylene glycol | 7.0 |
| 2-amino-2-methyl-1,3-propanediol | 5.0 |
| glutamic acid | 0.7 |
| ethyl paraben | 0.3 |
| perfume | suitable amount |
| purified water | balance |

### 〈Preparation Method〉

The mixture of 2-amino-2-methyl-1,3-propanediol, glutamic acid, and propylene glycol was added to the purified water. The mixture was maintained at 70°C (aqueous phase). The remaining ingredients were mixed, and the mixture was maintained at 70°C (oily phase). The oily phase was continuously stirred and the aqueous phase was slowly added thereto. The mixture was emulsified by use of a homogenizer. The thus-obtained emulsion was cooled to 30°C under sufficient stirring to obtain a milky lotion.

| [Formulation Example 16] | Jelly |
|---|---|
| Ingredient | Amount (wt.%) |
| 95% ethyl alcohol | 10.0 |
| dipropylene glycol | 15.0 |
| POE (50 mol) oleyl ether | 2.0 |
| carboxyvinyl polymer (trade name: Carbopole 940, B. F. Goodrich Chemical Company) | 0.05 |
| 2-amino-2-methyl-1,3-propanediol | 0.1 |
| gluconic acid | 0.1 |
| ethyl paraben | 0.3 |
| perfume | suitable amount |
| purified water | balance |

### 〈Preparation Method〉

Carbopole 940 was dissolved thoroughly in the purified water. The POE (50 mol) oleyl ether was added to the resultant aqueous phase. Subsequently, the resultant aqueous phase was neutralized and thickened through the addition of the 2-amino-2-methyl-1,3-propanediol, to which the remaining ingredients had been added in advance, to obtain a jelly.

| [Formulation Example 17] | Jelly |
|---|---|
| Ingredient | Amount (wt.%) |
| (phase A) | |
| 95% ethyl alcohol | 10.0 |
| POE (20 mol) octyldodecanol | 1.0 |
| pantothenyl ethyl ether | 0.1 |
| methyl paraben | 0.15 |

| (phase B) | |
|---|---|
| potassium hydroxide | 0.1 |

| (phase C) | |
|---|---|
| glycerin | 5.0 |
| dipropylene glycol | 10.0 |
| 2-amino-2-methyl-1,3-propanediol hydrochloride | 0.05 |
| carboxyvinyl polymer (trade name: Carbopole 940, B. F. Goodrich Chemical Company) | 0.2 |
| purified water | balance |

### 〈Preparation Method〉

The "phase A" ingredients were homogenously mixed. Similarly, the "phase C" ingredients were homogenously mixed The thus-obtained "phase A" mixture was added to and dissolved in the "phase C" mixture. The "phase B" ingredient was added thereto and the resultant mixture was charged into a container to obtain a jelly.

| [Formulation Example 18] | Pack |
|---|---|
| Ingredient | Amount (wt.%) |
| (phase A) | |
| dipropylene glycol | 5.0 |
| POE (60 mol) hydrogenated castor oil | 5.0 |

| (phase B) | |
|---|---|
| olive oil | 5.0 |
| tocopheryl acetate | 0.2 |
| ethyl paraben | 0.2 |
| perfume | 0.2 |

| (phase C) | |
|---|---|
| 2-amino-2-methyl-1,3-propanediol sulfate | 0.3 |
| polyvinyl alcohol (saponification value: 90; polymerization degree: 2,000) | 13.0 |
| ethyl alcohol | 7.0 |
| purified water | balance |

### 〈Preparation Method〉

Each of the "phase A", "phase B", and "phase C" ingredients were homogenously mixed. The "phase B" mixture was added to and dissolved in the "phase A" mixture. Subsequently, the "phase C" mixture was added thereto, and the resultant mixture was charged into a container, to obtain a pack.

| [Formulation Example 19] | Solid-type foundation |
|---|---|
| Ingredient | Amount (wt.%) |
| talc | 43.1 |
| kaolin | 15.0 |
| sericite | 10.0 |
| zinc oxide | 7.0 |
| titanium dioxide | 3.8 |
| yellow iron oxide | 2.9 |
| black iron oxide | 0.2 |
| squalane | 8.0 |
| isostearic acid | 4.0 |
| POE sorbitan monooleate | 3.0 |
| isocetyl octanoate | 2.0 |
| 2-amino-2-methyl-1,3-propanediol | 0.2 |
| lactic acid | 0.5 |
| preservative | suitable amount |
| perfume | suitable amount |

### 〈Preparation Method〉

The powdery ingredients from the talc to the black iron oxide were sufficiently mixed by use of a blender. The mixture of the oily ingredients from the squalane to the isocetyl octanoate, the 2-amino-2-methyl-1,3-propanediol, the lactic acid, the preservative, and the perfume was added to the previous mixture. The mixture was thoroughly kneaded, charged into a container, and then shaped, to obtain a solid-type foundation.

| [Formulation Example 20] | Emulsion-type foundation |
|---|---|
| Ingredient | Amount (wt.%) |
| (powdery ingredients) | |
| titanium dioxide | 10.3 |
| sericite | 5.4 |
| kaolin | 3.0 |
| yellow iron oxide | 0.8 |
| red iron oxide | 0.3 |
| black iron oxide | 0.2 |

| (oily phase) | |
|---|---|
| decamethylcyclopentasiloxane | 11.5 |
| liquid paraffin | 4.5 |
| POE-modified dimethylpolysiloxane | 4.0 |

| (aqueous phase) | |
|---|---|
| purified water | 50.0 |
| 1,3-butylene glycol | 4.5 |
| 2-amino-2-methyl-1,3-propanediol | 1.5 |
| sorbitan sesquioleate | 3.0 |
| preservative | suitable amount |
| perfume | suitable amount |

### 〈Preparation Method〉

The aqueous phase ingredients were mixed and heated with stirring. The powdery ingredients which had been thoroughly mixed and pulverized were added thereto and subjected to processing in a homogenizer. The oily phase ingredients which had been heated and mixed were added, and the resultant mixture was processed by use of a homogenizer. The perfume was added under stirring, followed by cooling to room temperature, to obtain an emulsion-type foundation.

## Claims

1. An external-use composition comprising a propanediol derivative represented by formula (I): 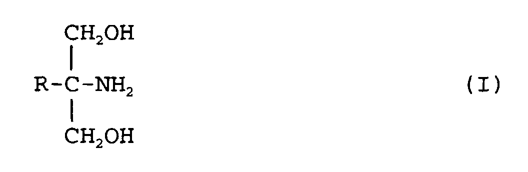 (wherein R represents a hydrogen atom, a methyl group, or an ethyl group) or a pharmaceutically acceptable salt thereof.

2. An external-use composition according to claim 1, wherein the propanediol derivative represented by formula (I) is 2-amino-1,3-propanediol.

3. An external-use composition according to claim 1, wherein the propanediol derivative represented by formula (I) is 2-amino-2-methyl-1,3-propanediol.

4. An external-use composition according to claim 1, wherein the propanediol derivative represented by formula (I) is 2-amino-2-ethyl-1,3-propanediol.

5. An external-use composition according to claim 1, wherein the amount of the propanediol derivative represented by formula (I) is 0.005-20.0 wt.% with respect to the entirety of the external-use composition.

6. An external-use composition according to claim 1, which exhibits retardation action against aging of the skin.

7. An external-use composition according to claim 1, which exhibits preventive action against spots.

8. An external-use composition according to claim 1, which exhibits preventive action against dull appearance.

9. An external-use composition according to claim 1, which exhibits preventive action against wrinkles.

10. An external-use composition according to claim 1, which exhibits preventive action against skin-roughening.
